# EUROPEAN PATENT SPECIFICATION

(11) **EP 1 063 229 B1**
(45) Date of publication and mention of the grant of the patent: **19.02.2003**
(21) Application number: 00105851.0
(22) Date of filing: 20.03.2000
(51) Int. Cl.: C07C 403/16, C07C 49/21, C07C 49/557, C11B 9/00, A61K 7/46

(54) **Alpha, beta-unsaturated ketones**
Alpha, beta-ungesättigte Ketone
Cétones alpha, béta-insaturées

(30) Priority: 27.05.1999 EP 99810461
(43) Date of publication of application: 27.12.2000
(73) Proprietor: Givaudan SA, 1214 Vernier-Genève (CH)
(72) Inventor: Berg-Schultz, Katja, 8064 Zürich (CH); Bajgrowicz, Jerzy A., 8053 Zürich (CH)
(74) Representative: Patentanwälte Schaad, Balass, Menzl & Partner AG

(56) References cited:
- US-A- 4 147 672
- US-A- 4 372 881
- US-A- 5 504 066
- MASSO, TERESA ET AL: "The Claisen transposition in the preparation of new odorant substances" PERFUM. FLAVOR. (1990), 15(4), 39-40, 42-3 , XP000957533

## Description

The present invention relates to α,β-unsaturated ketones exhibiting a green-galbanum, fresh (metallic, undecatriene type) and fruity-pineapple and/or fruity-cassis odor and to the use of these compounds as fragrance materials in different compositions.

Inexpensive floral fruity and green fragrances with intense pineapple and galbanum undertones are highly desirable in the art of perfumery. However, many of the natural and commercially available compounds e.g. galbanum oil are expensive and show lack of stability. Moderately weak galbanum type compounds became available in the mid seventies including allyl amyl glycolate (International Flavor and Fragrance Inc.) and Cyclogalbanate® (Dragocco S.A.). Another member of this family, the allyl cyclopentyl glycolate is described in the US 4 735 932. However, glycolates are generally not stable in alkali, acid or oxidizing media.

1- (5,5-dimethyl-1-cyclohexen-1-yl)-4-penten-1-one is described by Morris, A.F.; Näf, F; Snowdon, R. L. in Perfumer & Flavorist **1991** (16), 33 as an important compound for the perfumery. This compound possesses a powerful metallic odor reminiscent of galbanum with pineapple and hyacinth character that adds fresh, green, floral and fruity aspects to perfumes and perfumed products.

The object of the present invention is to provide compounds which have an intense and long lasting green galbanum type of odor accompanied by various fruity, mainly pineapple undertones.

It has surprisingly been found that compounds of Formula I 1-substituted-4-penten-1-one derivatives useful as fragrance materials have also been disclosed in US-A-4 147 672, US-A-4 372 881 and by Masso' T in Perfumer & Flavorist 1990 (15), 39. 2,2,3-trimethyl-3-cyclopenten-1-yl derivatives useful as fragances have been disclosed in US-A-5 504 066. 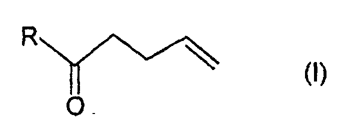 wherein R is a residue A 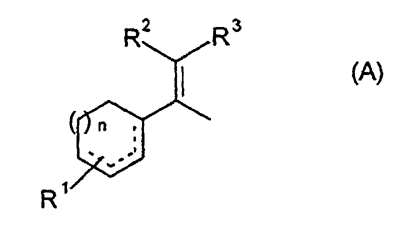 or a residue B 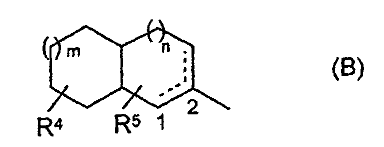 and R¹, R², R³, R⁴ and R⁵ are independently hydrogen, methyl or ethyl, R¹, R⁴ and R⁵ being at any position of the ring structure, n and m are independently 0,1,2 or 3 and the dotted lines stand in formula A, if desired, for a double bond and the dotted lines in formula B stand, for a double bond either in position 1 or 2, have an intense, very long lasting green-galbanum type of odor with fruity undertones. These characteristics make the compounds well suited for difficult functional perfumery applications, as well as for imparting unique green galbanum and fruity notes to fine perfumery products.

The above formulas include all different possible stereo- and double-bond isomers.

Compounds of formula Ia are preferred. 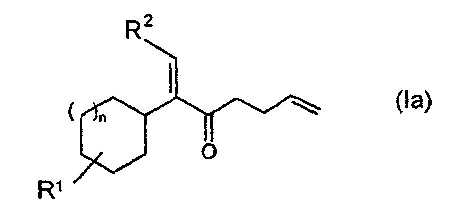 wherein R¹ and R² are independently hydrogen or methyl and n stands for 0 or 1.

Further preferred compounds are:
2-cyclohexylhepta-1,6-dien-3-one
(E)- and (Z)-3-cyclohexylocta-2,7-dien-4-one
2-cyclopentylhepta-1,6-dien-3-one
1-(3,4,4a,5,6,7,8,8a-octahydronaphth-2-yl)pent-4-en-1-one
1-(1,4,4a,5,6,7,8,8a-octahydronaphth-2-yl)pent-4-en-1-one

In addition to the above excellent qualities, it has been found that the compounds of the invention show outstanding diffusion and/or high substantivity, the latter meaning persistence of odor. The high diffusion and substantivity is well perceived on fabrics washed with a detergent or treated with a softener comprising one or more of the new α,β-unsaturated ketones. The typical fresh green odor is already perceived very strongly on the wet fabric and later also on the dry material.

Due to the excellent odor and application qualities, the compounds are excellent fragrances for use in any field of fine and functionary perfumery, such as perfumes, household products, laundry products, body care products and cosmetics.

The α,β-unsaturated ketones of formula I, wherein R is a residue of formula A, may be prepared according to procedures known in the art as shown in Scheme 1.

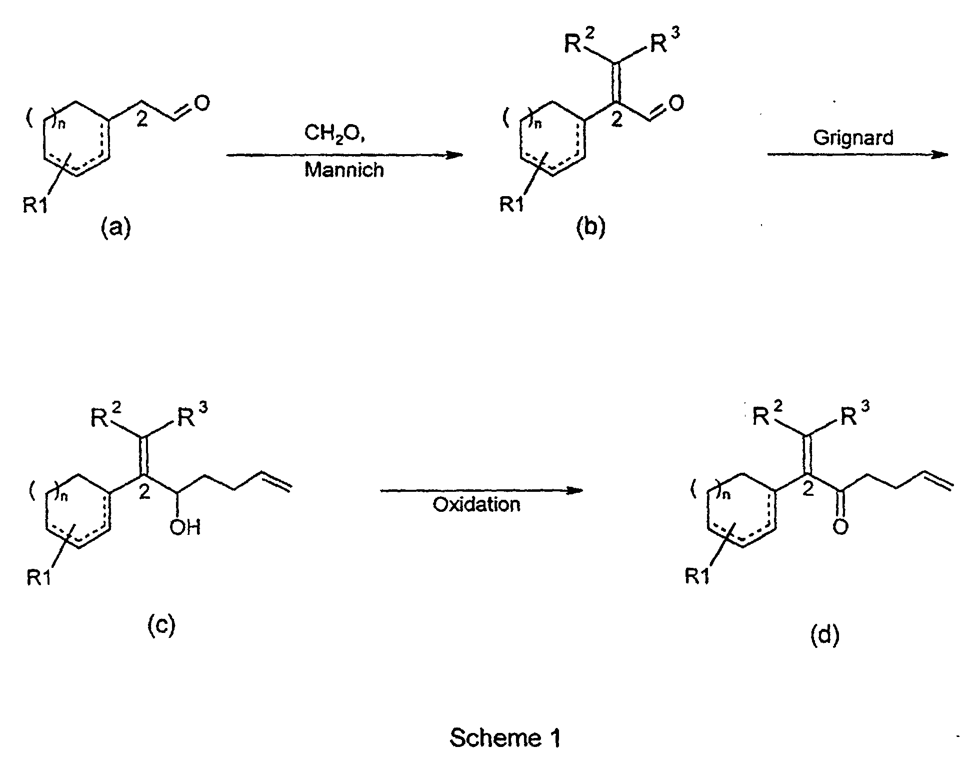

The starting materials of formula (a) in Scheme 1 may be prepared by oxidation of the corresponding alcohols. By methylenation with formaldehyde (Mannich reaction) aldehydes of formula (b), wherein R² and R³ are hydrogen are obtained.

The α,β-unsaturated aldehydes corresponding to compounds of Formula I wherein R is a residue of formula A and R² and/or R³ are not hydrogen may be for example prepared via cross-aldol condensation using Mukaiyama conditions followed by dehydration (T. Mukaiyama, K. Banno, K. Narasaka: J. Amer. Chem. Soc. **1974** (96), 7503).

Alternatively, the Grignard reaction may be carried out using appropriately substituted nitriles. This one pot reaction gives good yields of α,β-unsaturated ketones. 2-Substituted-3-trimethylsilyloxypropionitriles are especially well suited for this transformation. The latter can for example be prepared from the respective cyclic ketones and ethyl cyanoacetate according to J.A. Marshall, R.D. Carroll: J. Org. Chem. **1965** (30), 2748 followed by protection of the hydroxy-group with trimethylchlorosilane.

Another possible preparation of the α,β-unsaturated ketones of Formula I in which R is a residue A proceeds via Claisen transposition of the corresponding vinyl ether in analogy to T. Masso, A. Portella, E. Rus (in Perfumer & Flavorist **1990** (15), 39) starting with the suitably substituted 3-butene-2-ones (e.g. prepared according to R.A. Cormier, W.L. Schreiber, W.C. Agosta: J. Amer. Chem. Soc. **1973** (95), 4873).

The α,β-unsaturated ketones of Formula I wherein R is a residue of formula B can be prepared from the corresponding bicyclic ketones according to the process illustrated in Scheme 2.

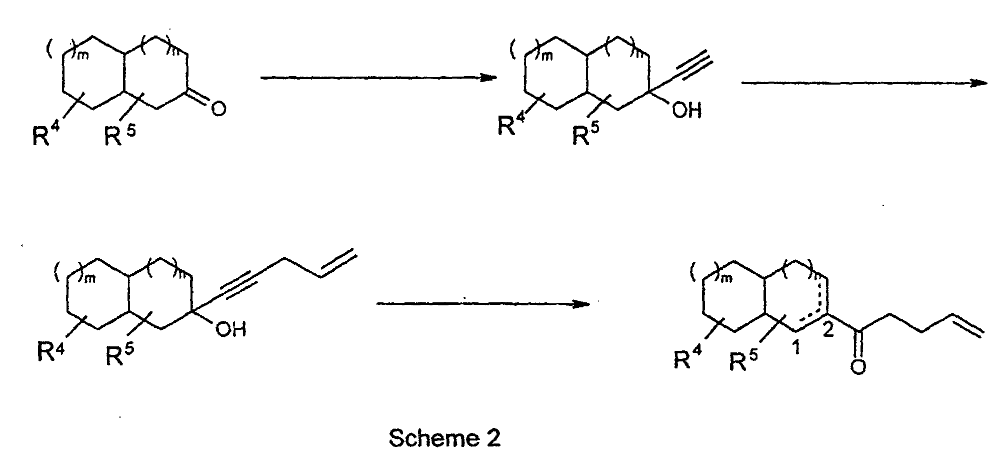

The starting ketones are either commercially available, or can be synthetized by Robinson annullation from the corresponding ketones with substituted or unsubstituted methyl vinyl ketones, followed by selective hydrogenation with Pd/C (e.g. G. Stork, A. Brizzolara, H. Landesman, J. Szmuskovicz, R. Terrell: J. Amer. Chem. Soc **1963** (85), 207).

The α,β-unsaturated ketones wherein R is a residue of formula B, are generally obtained as mixtures of cis-/trans-isomers. The double bond is either in position 1 or 2. The odors of the different isomers are all of the same "green" type, however the GC thresholds are varying within broad ranges. Cis-1-(1,4,4a,5,6,7,8,8a-octahydronaphth-2-yl)pent-4-ene-1-one, the most powerful isomer of n=m=1, R⁴=R⁵=H, has a GC-threshold of only 10 pg/l.

The odorants of the present invention may be combined with numerous odorant ingredients of natural and/or synthetic origin. The range of the natural odorants includes in addition to readily volatile, also moderately and only slightly volatile components. The synthetic odorants embrace representatives from practically all classes of odorant substances. The following list comprises examples of known odorants which may be combined with the compounds of the invention:
natural products: tree moss absolute, basil oil, tropical fruit oils (such as bergamot oil, mandarin oil, etc.), mastix absolute, myrtle oil, palmarosa oil, patchouli oil, petitgrain oil, wormwood oil, lavender oil, rose oil, jasmin oil, ylang-ylang oil, etc.;
alcohols: farnesol, geraniol, linalool, nerol, phenylethyl alcohol, rhodinol, cinnamic alcohol, (Z)-hex-3-en-1-ol, menthol, α-terpineol, etc.;
aldehydes: citral, α-hexyl cinnamaldehyde, Lilial, methylionone, verbenone, nootkatone, geranylacetone, etc.;
esters: allyl phenoxyacetate, benzyl salicylate, cinnamyl propionate, citronellyl acetate, decyl acetate, dimethylbenzylcarbinyl acetate, dimethylbenzylcarbinyl butyrate, ethyl acetoacetate, cis-3-hexenyl isobutyrate, cis-3-hexenyl salicylate, linalyl acetate, methyl dihydrojasmonate, styralyl propionate, vetiveryl acetate, benzyl acetate, geranyl acetate, etc.;
lactones: γ-undecalactone, δ-decalactone, pentadecanolide, 12-oxahexadecanolide, etc.;
acetals: Viridine (phenylacetaldehyde dimethylacetal), etc.;
other components often used in perfumery: indole, p-mentha-8-thiol-3-one, methyleugenol, eugenol, anethol, etc..

The novel compounds of the invention harmonize particularly well with floral notes (lily of the valley, rose, iris, jasmine, ylang-ylang, narcissus notes, etc.) as well as with woody, chypre and animalic notes, tobacco like an patchouli compositions, etc.

The percentage in which the compounds of the invention are used in a composition may vary within wide limits ranging from a few parts per thousand in mass market products (e.g. cleaning compositions, deodorant, etc.) up to a few percents in alcoholic extracts for fine perfumery. In all cases, even in small amounts, the compounds of Formula I provide odorant compositions with green-galbanum and intense fresh green-fruity notes and a remarkable increase of the volume (strength, diffusivity) and of the duration (substantivity) of the odor.

There is no restriction regarding the type of formulation. and the destination of the actual finished product: thus, eau de cologne, toilet water, scented water, perfume, body care and cosmetic products such as cream, shampoo, soap, household products such as detergent, household cleaner, fabric softener, etc., come into consideration.

The invention will be further described, by way of illustration, in the following examples.

All compounds were unambiguously identified by their ¹H-NMR-(chemical shifts (δ) are given in ppm downfield from TMS; coupling constants *J* in Hz),IR- and MS-spectra.

### Compounds of Formula I and R = A

### Example 1

### 2-Cyclohexylhepta-1,6-dien-3-one

### a) 2-Cyclohexylprop-2-en-1-al

Successively, sulfuric acid 62% (79.4 g, 0.5 mol), aqueous formaldehyde 35% (82 g, 1.0 mol) and cyclohexyl acetaldehyde (100.8 g, 0.8 mol) were added slowly to diethylamine (73 g, 1.0 mol) at 0°C. After stirring at 80°C overnight, the reaction mixture was extracted with MTBE. The combined organic phases were washed with H₂O until neutral pH, dried (MgSO₄) and concentrated in vacuo. The crude product was distilled under reduced pressure using a Widmer column (10 Torr, 80°C) yielding 89.5 g (81 %) of pure product as colorless oil. ¹H-NMR (200MHz, CDCl₃): 9.5 (*s*, 1H, -CHO), 6.22 (*s*, 1H, H-C(3)), 5.95 (*s*, 1H, H'-C(3)), 2.48 (*m*, 1H, -CH-), 1.9-1.6 (*m*, 5H, -CH₂-), 1.5-1.0 (*m*, 5H, -CH₂-). MS (EI): 138(M⁺,40), 123(22), 109(73), 95(90), 91(28), 79(61), 67(100), 55(39), 41(53). IR (neat): 2927*vs*, 2853 *s*, 1694*vs*, 1449*m* cm⁻¹.

### b) 2-Cyclohexylhepta-1,6-dien-3-ol

2-Cyclohexylprop-2-en-1-al (69 g, 0.5 mol) in 100 ml of ether was slowly added to but-1-enylmagnesium bromide prepared by slow addition of 4-bromo-but-1-ene (81.0 g, 0.6 mol) in 100 ml of ether at 0°C to magnesium turnings (13.2 g, 0.55 mol) in 300 ml of ether. After the addition was complete, the reaction mixture was allowed to warm to RT and was stirred for additional 3h. After quenching with 2N HCl the organic phase was separated, washed until neutral pH with H₂O, dried (MgSO₄) and the solvent was evaporated in vacuo. The crude product was distilled over a Vigreux column (0.15 Torr, 91°C) yielding 85 g (88%) of pure product as colorless oil. ¹H-NMR (200MHz, CDCl₃): 5.85 (*ddt*, *J* = 17.2, 10.4, 6.6, 1H, H-C(6)), 5.05 (*ddt*, *J* = 17.2, 2.0, 1.6, 1H, H-C(7)), 5.02 (*dd*, *J* = 1.0, 1.0, 1H, H-C(1)), 4.98 (*ddt*, *J* = 10.2, 2.1, 1.2, 1H, H'-C(7)), 4.88 (*d*, *J* = 1.0, 1H, H'-C(1)), 4.09 (*t*, *J* = 6.2, 1H, H-C(3)), 2.22-2.10 (*m*, 2H, H-C(5)), 1.89 (*m*, 1H, -CH-), 1.80-1.52 (*m*, 8H, -CH₂-, -OH), 1.38-1.24 (*m*, 5H, -CH₂-). MS (EI) : 194(M⁺,1), 176(4), 165(3), 151(9), 139(11), 122(11), 109(15), 95(37), 83(66), 79(43), 71(34), 67(50), 55(100), 41(50). IR (neat): 3361*s*, 2926*vs*, 2852*s*, 1641*w*, 1448*m* cm⁻¹.

### c) 2-Cyclohexylhepta-1,6-dien-3-one

2-Cyclohexyl-hepta-1,6-dien-3-ol (85 g, 0.44 mol) were oxidized with MnO₂ (804 g, 9.2 mol) in 2 1 of hexane at RT. After stirring for 96 h the reaction mixture was filtered over Celite and concentrated in vacuo yielding 71 g of a yellow oil. Distillation (0.04 Torr/ 70°C) yielded 43.5 g (51%) of pure product.

Odor: green-galbanum, fruity-pineapple, metallic.
¹H-NMR (400MHz, CDCl₃): 5.95 (*s*, 1H, H-C(1)), 5.85 (*ddt*, *J* = 17.0, 10.2, 6.4, 1H, H-C(6)), 5.65 (*d*, *J* = 1.2, 1H, H'-C(1)), 5.05 (*ddt*, *J* = 17.1, 1.6, 1.6, 1H, H-C(7)), 4.96 (*ddt*, *J* = 10.2, 1.6, 1.2, 1H, H'-C(7)), 2.78 (*t*, *J* = 7.4, 2H, H-C(4)), 2.59 (*m*, 1H, -CH-), 2.35 (*m*, 2H, H-C(5)), 1.80-1.65 (*m*, 5H, -CH₂-), 1.41-1.28 (*m*, 2H, -CH₂-), 1.22-1.00 (*m*, 3H, -CH₂-). MS (EI): 192(M⁺,11), 177(4), 163(50), 149(29), 137(78), 121(9), 109(65), 95(22), 81(34), 77(12), 67(100), 55(61), 41(29). IR (neat) : 2926*vs*, 2852*s*, 1681*vs*, 1449*m* cm⁻¹.

The following examples are prepared according to the general procedure described for example 1. Only the spectroscopic data and olfactory properties are given below.

### Example 2

### 2-Cyclopentylhepta-1,6-dien-3-one

Odor: green-galbanum, anisic, metallic
¹H-NMR (400MHz, CDCl₃): 5.95 (*s*, 1H, H-C(1)), 5.85 (*ddt*, *J* = 17.2, 10.4, 6.6, 1H, H-C(6)), 5.70 (*d*, *J* = 1.2, 1H, H'-C(1)), 5.04 (*ddt*, *J* = 17.1, 1.8, 1.6, 1H, H-C(7)), 4.98 (*ddt*, *J* = 10.2, 1.6, 1.2, 1H, H'-C(7)), 2.95 (*m*, 1H, -CH-), 2.80 (*t*, *J* = 7.5, 2H, H-C(4)), 2.40 (*m*, 2H, H-C(5)), 1.94-1.81 (*m*, 2H, -CH₂-), 1.75-1.55 (*m*, 4H, -CH₂-), 1.35-1.12 (*m*, 2H, -CH₂-). MS (EI): 178(M⁺,1), 163(2), 149(18), 137(5), 123(43), 110(8), 95(100), 83(9), 79(15), 67(73), 55(61), 41(23). IR (neat): 2953*vs*, *2869s,* 1681*vs*, 1641*m* cm⁻¹.

### Example 3

### 2-(4-Methylcyclohexyl)hepta-1,6-dien-3-one (cis/trans isomers ~1:1)

Odor: green-galbanum, fruity-pineapple
¹H-NMR (400MHz, CDCl₃): 5.96 + 5.95 (2*s*, 1H, H-C(1)), 5.83 (*ddt*, *J* = 17.2, 10.4, 6.6, 1H, H-C(6)), 5.69 + 5.65 (2*d*, *J* = 1.4, 1H, H'-C(1)), 5.00 (*m*, 2H, H-C(7)), 2.77 (*t*, *J* = 7.6, 2H, H-C(4)), 2.55 (*m*, 1H, -CH-), 2.35 (*m*, 2H, H-C(5)), 1.94-1.22 (*m*, 7H,-CH-, -CH₂-), 1.19-1.00 (*m*, 2H, -CH₂-), 1.00 + 0.89 (2*d*, *J* = 7.2, 6.4, 3H, -CH₃). MS (EI): 206 (M⁺,4), 191(6), 177(44), 163(7), 151(44), 149(33), 123(22), 110(17), 109(17), 107(14), 105(10), 95(34), 81(100), 79(24), 67(42), 55(65), 41(28). IR (neat): 2922*vs*, 2850*s*, 1681*vs*, 1641*m* cm⁻¹.

### Example 4

### 2-(3-Methylcyclohexyl)hepta-1,6-dien-3-one (cis/trans isomers ∼1:1)

Odor: green-fruity, pineapple with galbanum-aspects
¹H-NMR (400MHz, CDCl₃): 5.96 + 5.95 (2*s*, 1H, H-C(1)), 5.83 (*ddt*, *J* = 16.8, 10.4, 6.6, 1H, H-C(6)), 5.70 (*2d*, *J* = 1.2, 1H, H'-C(1)), 5.00 (*m*, 2H, H-C(7)), 2.9 (*m*, 0.5H, -CH-), 2.77 (*m*, 2H, H-C(4)), 2.61 (*m*, 0.5H, -CH-), 2.46 (*m*, 2H, H-C(5)), 2.01-1.09 (*m*, 9H, -CH-, -CH₂-), 1.03 + 0.88 (2*d*, *J* = 7.2, 6.8, 3H, -CH₃). MS (EI): 206(M⁺,4), 191(6), 177(30), 163(18) 151(49), 149(11), 123(20), 110(22), 109(15), 107(13), 105(8), 95(33), 81(100), 79(22), 67(42), 55(60), 41(28). IR (neat): 2923*vs*, 2850*s*, 1681*vs*, 1641*m* cm⁻¹.

### Example 5

### 2-(2-Methylcyclohexyl)hepta-1,6-dien-3-one (cis/trans isomers ∼1:1)

Odor: green-galbanum, mushroom, fruity-pineapple with jasmin aspects
¹H-NMR (400MHz, CDCl₃): 6.06 + 6.02 (2*s*, 1H, H-C(1)), 5.82 (*m*, 1H, H-C(6)), 5.70 + 5.52 (2*d*, *J* = 1.2, 1H, H'-C(1)), 5.00 (*m*, 2H, H-C(7)), 2.80 (*m*, 2.5H, H-C(4), -CH-), 2.38 (*m*, 2H, H-C(5)), 1.95 (*m*, 0.5H, -CH-), 1.82-1.00 (*m*, 9H,-CH-, -CH₂-), 0.71 + 0.69 (2*d*, *J* = 6.4, 7.2, 3H, -CH₃). MS (EI) : 206(M⁺,15), 191(10), 177(33), 164(23) 151(50), 149(32), 133(124), 123(27), 109(28), 107(22), 105(22), 95(49), 81(89), 79(35), 67(51), 55(100), 41(44). IR (neat): 2925*vs*, 2853*s*, 1681*vs*, 1641*m* cm⁻¹.

### Example 6

### 2-(2-Methylcyclopentyl)hepta-1,6-dien-3-one (cis/trans isomers)

Odor: green-galbanum, pineapple, grapefruit with anisic aspects
¹H-NMR (400MHz, CDCl₃): 6.08 + 6.01 (2*s*, 1H, H-C(1)), 5.82 (*m*, 1H, H-C(6)), 5.70 + 5.66 (2*d*, J = 1.2, 1H, H'-C(1)), 5.02 (*m*, 2H, H-C(7)), 3.10 - 1.20 (5*m*, 12H), 0.90 + 0.54 (2*d*, *J*= 6.4, 6.8, 3H, -CH₃). MS (EI) : 192(M⁺,4), 177(14), 163(68), 150 (23) , 137(63), 133(124), 123(11), 119(25), 109(63), 107(23), 105(9), 95(37), 81(56), 79(33), 67(95), 55(100), 41(47). IR (neat): 2954*vs*, 2869*s*, 1681*vs*, 1641*m* cm⁻¹.

### Example 7

### 2-Cycloheptylhepta-1,6-dien-3-one

Odor: green-galbanum, fruity, floral (lindenblossom)
¹H-NMR (400MHz, CDCl₃): 5.95 (*s*, 1H, H-C(1)), 5.85 (*ddt*, *J* = 17.2, 10.4, 6.4, 1H, H-C(6)), 5.68 (*d*, *J* = 1.2, 1H, H'-C(1)), 5.05 (*ddt*, *J* = 17.2, 1.6, 1.6, 1H, H-C(7)), 4.96 (*ddt*, *J* = 10.4, 1.6, 1.2, 1H, H'-C(7)), 2.77 (*t*, *J* = 7.2, 2H, H-C(4)), 2.72 (*m*, 1H, -CH-), 2.37 (*m*, 2H, H-C(5)), 1.74-1.29 (*m*, 12H, -CH₂-). MS(EI): 206(M⁺,3), 191(2), 177(27), 165(9), 151(38), 149(22), 135(6), 123(11), 121(13), 109(22), 95(30), 81(100), 67(96), 55(100), 41(51). IR (neat): 2924*vs*, 2855*s*, 1680*vs*, 1641*m* cm⁻¹.

### Example 8

### 2-Cyclooctylhepta-1,6-dien-3-one

Odor: green-galbanum, woody, fatty with marine aspects
¹H-NMR (400MHz, CDCl₃): 5.95 (*s*, 1H, H-C(1)), 5.85 (*ddt*, *J* = 17.1, 10.1, 6.6, 1H, H-C(6)), 5.69 (*d*, *J* = 1.1, 1H, H'-C(1)), 5.05 *(ddt, J* = 17.1, 1.7, 1.7, 1H, H-C(7)), 4.96 (*ddt*, *J* = 10.2, 1.6, 1.2, 1H, H'-C(7)), 2.96 (*m*, 1H, -CH-), 2.75 (*t*, *J* = 7.4, 2H, H-C(4)), 2.35 (*m*, 2H, H-C(5)), 1.80-1.45 (*m*, 14H, -CH₂-). MS(EI): 220(M⁺,4), 205(2), 191(30), 179(19), 177(14), 149(28), 137(15), 135(17), 123(21), 121(24), 109(32), 110(22), 95(94), 81(79), 67(54), 55(100), 41(47). IR (neat): 2920*vs*, 2851*s*, 1680*vs*, 1641*m* cm⁻¹.

### Example 9

### 3-Cyclohexylocta-2,7-dien-4-one

### a) 3-Cyclohexylbut-2-enal

A mixture of [(2-cyclohexylethenyl)oxy)]trimethylsilane (19.8 g, 0.1 mol) and acetaldehyde (4.4 g, 0.1 mol) was added slowly at -70°C to a mixture of titantetrachloride (11 ml, 0.1 mol) and titanium(IV) isopropoxide (0.59 ml, 0.02 mol) in CH₂Cl₂ (50 ml). After stirring for 1 h, the reaction was quenched with saturated NH₄Cl-solution and extracted twice with ether (2 x 100 ml). The combined organic phases were washed to neutral pH, dried (MgSO₄) and concentrated in vacuo. The crude product was dehydrated by distillation with I₂ (200 mg) over a 5 cm Widmer column (0.1 Torr, 120°C) yielding 7.7 g (50 %) of 3-cyclohexylbut-2-enal as E/Z-mixture of ∼7:1. ¹H-NMR ((*E*)-isomer, 200MHz, CDCl₃): 9.31 (*d*, *J* = 1.0, 1H, -CHO), 6.48 (*q*, *J* = 7.5, 1H, H-C(3)), 2.55 (*m*, 1H, -CH-), 2.05 (*d*, *J* = 7.5, 3H, CH₂), 2.00-1.05 (*m*, 10H, -CH₂-). MS (EI): 152(M⁺,72), 137 (29) , 134 (14), 123 (100) , 119(24), 109(43), 105(25), 95(57), 91(37), 84(21), 81(68), 79(45), 77(24), 69(31), 67(72), 55(48), 41(50). IR (neat): 2927*vs*, 2853*s*, 2704*w*, 1689*s*, 1634*m*, 1449*m* cm⁻¹.

### b) 3-Cyclohexylocta-2,7-dien-4-one (E/ Z mixture of 10:1)

was prepared following the experimental procedure described in example 1a and 1c.

Odor: green-galbanum, pineapple, cassis
¹H-NMR ((*E*)-isomer, 400MHz, CDCl₃): 6.50 (*q*, *J* = 7.0, 1H, H-C(2)), 5.80 (*ddt*, *J* = 16.8, 10.4, 6.4, 1H, H-C(7)), 5.01 (*ddt*, *J* = 17.1, 1.6, 1.6, 1H, H-C(8)), 4.96 (*ddt*, *J* = 10.2, 1.8, 1.2, 1H, H'-C(8)), 2.68 (*t*, *J* = 7.6, 2H, H-C(5)), 2.55 (*tt*, *J* = 12.2, 3.4, 1H, -CH-), 2.35 (*m*, 2H, H-C(6)), 1.88 (*d*, *J* = 6.8, -CH₃), 1.85-1.60 (*m*, 5H, -CH₂-), 1.50-1.10 (*m*, 5H, -CH₂-), MS (EI): 206(M⁺,10), 191(15), 177(16), 164 (9), 151 (100), 123(19), 109(8), 95(12), 81(80), 79(18), 67(43), 55(49), 41(20). IR (neat): 2926*vs*, 2852*s*, 1672*s*, 1641*m*, 1450*m* cm⁻¹.

### Example 10

### 4-Cyclohexylnona-3,8-dien-5-one (20:1 E/Z mixture)

was prepared according to the general procedure described for 3-cyclohexylocta-2,7-dien-4-one (Example 9).

Odor: green-fruity, apple, pineapple, galbanum, anisic
¹H-NMR ((*E*)-isomer, 400MHz, CDCl₃): 6.35 (*t*, *J* = 7.4, 1H, H-C(3)), 5.82 (*ddt, J* = 17.0, 10.4, 6.4, 1H, H-C(8)), 5.01 (*ddt, J* = 17.1, 1.8, 1.7, 1H, H-C(9)), 4.96 (*ddt*, *J* = 10.2, 1.8, 1.2, 1H, H'-C(9)), 2.70 (*t*, *J* = 7.5, 2H, H-C(6)), 2.51 (*tt*, *J* = 12.0, 3.6, 1H, -CH-), 2.39-2.30 (*m*, 2H, H-C(7)), 2.29 (*q*, *J* = 7.6, H-C(2)), 1.82-1.60 (*m*, 5H, -CH₂-), 1.45 (*m*, 2H, -CH₂-), 1.32-1.18 (*m*, 3H, -CH₂-), 1.08 (*t*, *J* = 7.6, -CH₃). MS (EI, (*E*)-isomer): 220(M⁺,10), 191(43), 177(9), 165 (100), 149 (5), 137(11), 123(7), 109(12), 95(86), 81(72), 79(23), 67 (34) , 55(63), 41(26). IR (neat): 2927*vs*, 2852*s*, 1672*s*, 1641*m*, 1450*m* cm⁻¹.

### Compounds of Formula I and R = B

### Example 11

### 1-(1,4,4a,5,6,7,8,8a-Octahydronaphth-2-yl)pent-4-ene-1-one 1-(3,4,4a,5,6,7,8,8a-Octahydronaphth-2-yl)pent-4-ene-1-one 1-(1,2,3,4,5,6,7,8,-Octahydronaphth-2-yl)pent-4-ene-1-one

### a) 2-Ethynyldecahydronaphthalen-2-ol

Acetylene was bubbled for 4h through a mixture of tBuOK (95.8 g, 0.85 mol) in THF (1 l) at 0°C. 2-Decalone (100 g, 0,66 mol) was added slowly at RT to the slightly yellow suspension and the resulting mixture was stirred for additional 3.5 h, quenched with saturated NH₄Cl (500 ml) solution and extracted with MTBE (2 x 700 ml). The combined organic phases were washed with NH₄Cl (500 ml), H₂O (2 x 500 ml), saturated NaCl (500 ml) solution until neutral pH, dried (MgSO₄) and concentrated in vacuo. The crude orange oil was flash distilled (0.01 Torr, 88-90°C) yielding 100.6 g (86%) of 2-ethynyldecahydro-naphthalen-2-ol.

### b) 2-(Pent-4-en-1-ynyl)decahydronaphth-2-ol

A solution of 2-ethynyldecahydronaphthalen-2-ol (100.6 g, 0.56 mol) in iPrOH (300 ml) was added slowly at 0°C under nitrogen atmosphere to a mixture of KOH ( 47.5 g), K₂CO₃ (6.5 g) and CuCl (4.4 g) in MeOH (300 ml). After stirring for additional 30 min at 0°C , allylbromide (102 g, 0.85 mol) was added slowly over a period of 25 min. The reaction mixture was stirred overnight at RT, quenched with NH₄Cl and concentrated in vacuo. The residue was taken up in saturated NH₄Cl (500 ml) solution, extracted with MTBE (2 x 400 ml) . The combined organic phases were washed with saturated NH₄Cl solution (500 ml), H₂O (2 x 500 ml), saturated NaCl solution (500 ml) until neutral pH, dried (MgSO₄) and evaporated in vacuo to yield quantitatively 120 g of crude product which was used without further purification in the next step.

### c) 1-(1,4,4a,5,6,7,8,8a-Octahydronaphth-2-yl)pent-4-ene-1-one 1-(3,4,4a,5,6,7,8,8a-Octahydronaphth-2-yl)pent-4-ene-1-one 1-(1,2,3,4,5,6,7,8,-Octahydronaphth-2-yl)pent-4-ene-1-one

A solution of 2-(pent-4-en-1-ynyl)decahydronaphalen-2-ol (120 g, 0.55 mol) in HCOOH 80% (200 ml) was heated at 90°C for 16 h. After cooling to RT, the resulting brown mixture was neutralized with saturated Na₂CO₃ and extracted with MTBE (2 x 400 ml). The combined organic phases are washed with NaHCO₃ (2 x 400 ml), H₂O (2 x 400 ml) and dried over MgSO₄. After distillation with a Vigreux column, 54.2 g (45%) of the olfactorily pure product was obtained as a mixture of 5 isomers.

Odor (mixture of 5 isomers): green-galbanum, fruity
¹H-NMR (200MHz, CDCl₃): 6.90 (*m*, ∼0.9H), 6.85 (*m*, 1H), 5.00 (*m*, 2H), 2.82-1.20 (*m*, 18H). MS (EI): Peak 1 (22%): 218(M⁺,18), 176(6), 163(30), 135(100), 119(7), 107(9), 105(8), 93(23), 91(38), 79(22), 67(24), 55(33), 41(12); Peak 2 (8%): 218(M⁺,55), 177(54), 163(100), 145(19), 135(92), 119(13), 107(24), 105(18), 93(47), 91(57), 79(49), 67(68), 55(54), 41(25); Peak 3 (15%): 218(M⁺,46), 177(50) , 163(87), 145(19), 135(100), 119(12), 107(24), 105(16), 93(47), 91(50), 79(47), 67(67), 55(47), 41(27); Peak 4 (13%): 218(M⁺,11), 177(1), 163(100), 145(2), 135(11), 107(7), 93(25), 91(15), 79(20), 67(20), 55(18), 41(11); Peak 5 (42%): 218(M⁺,8), 177(2), 163(100), 145(4), 135(11), 107(12), 93(24), 91(16), 79(21), 67(21), 55(20), 41(11). IR (mixture of 5 isomers, neat): 2923*s*, 2853*m*, 1669*vs*, 1640*m* cm⁻¹.

The main and most powerful isomer, cis-1-(1,4,4a,5,6,7,8,8a-octahydronaphth-2-yl)pent-4-ene-1-one, with a GC-odor threshold of 10 pg was isolated by preparative GLC for ¹H-NMR analysis. ¹H-NMR (400MHz, CDCl₃): 6.85 (*m*, 1H, C*H*=C), 5.85 (*ddt*, 1H, *J* = 17.0, 10.2, 6.6, H-C(4)), 5.05 (*ddt*, *J* = 17.1, 1.6, 1.6, 1H, H-C(5)), 4.98 (*ddt*, *J* = 10.1, 1.8, 1.2, H'-C(5)), 2.74 (*t*, *J* = 7.6, H-C(2)), 2.35 (*m*, 2H, -CH₂-), 2.30-2.13 (*m*, 4H, -CH₂-), 1.85 (*m*, 2H, -CH-), 1.57 (*m*, 2H, -CH₂-), 1.47-1.32 (*m*, 6H, -CH₂-).

The compounds of the following examples were all prepared according to the general procedure described in Example 11. Only the spectroscopic data and olfactory properties for each example are given below.

### Example 12

### 1-(2,3,3a,4,7,7a-hexahydro-1H-indene-5-yl)pent-4-ene-1-one 1-(2,3,3a,6,7,7a-hexahydro-1H-indene-5-yl)pent-4-ene-1-one 1-(2,3,4,5,6,7-hexahydro-1H-indene-5-yl)pent-4-ene-1-one

Odor (mixture of 3 isomers): green-galbanum, cassis, boysenberry, metallic
¹H-NMR (200MHz, CDCl₃): 6.90 (*m*, ∼0.8H), 6.85 (*m*,1H), 5.00 (*m*, 2H), 2.82-1.20 (*m*, 16H) . MS (EI): Peak 1 (16%) : 204(M⁺,18), 162(7), 149(34), 121(100), 107(5), 105(7), 93(25), 91(37), 79(39), 67(14), 55(35), 41(11); Peak 2 (61%): 204(M⁺,14), 163(16), 149(100), 131(17), 121(22), 107(13), 105(11), 93(25), 91(23), 79(34), 67(12), 55(18), 41(10); Peak 3 (23%): 204(M⁺,7), 163(2), 149(100), 131(6), 121(7), 107(5), 105(7), 93(26), 91(16), 79(32), 67(13), 55(19), 41(9). IR (mixture of 3 isomers, neat): 2943*s*, 2867*m*, 1710*w*, 1668*vs*, 1639*m* cm⁻¹.

### Example 13

### 1-(4a-Methyl-3,4,4a,5,6,7,8,8a-octahydronaphth-2-yl)pent-4-ene-1-one

### 1-(4a-Methyl-1,4,4a,5,6,7,8,8a-octahydronaphth-2-yl)pent-4-ene-1-one

Odor (mixture of 4 isomers): violet, woody, green-galbanum, pineapple
¹H-NMR (200MHz, CDCl₃): 6.85 + 6.75 (2*m*, 1H, CH=C), 5.85 (*m*, 1H, C*H*=CH₂), 5.00 (*m*, 2H, CH=C*H*₂), 2.75 (*m*, 2H), 2.60-1.00(*m*, 15H), 0.95 + 0.89 + 0.78 (3*s*, 3H). MS (EI): Peak 1 (32%) : 232(M⁺,56), 217(32), 204(3), 191(44), 177(100), 159(20), 149(26), 135(18), 121(16), 109(84), 96(23), 93(24), 91(55), 81(52), 79(39), 67(65), 55(68), 41(30); Peak 2 (52%) : 232(M⁺,15), 217(5), 204(3), 191(2), 177(100), 159(3), 149(16), 137(7), 121(3), 109(9), 107(9), 96(10), 93(13), 91(16), 81(73), 67(14), 55(23), 41(10); Peak 3 (2%): 232(M⁺,46), 217(25), 191(35), 177(43), 161(12), 159(11), 149(100), 135(13), 121(12), 109(70), 107(27), 93(42), 91(48), 81(52), 67(55), 55(67), 41(30); Peak 4 (14%): 232(M⁺,8), 217(3), 204(2), 191(2), 177(100), 159(3), 149(8), 121(5), 109(7), 107(16), 93(16), 91(12), 81(34), 67(14), 55(20), 41(10); IR (mixture of 4 isomers, neat): 2925*vs*, 2860*s*, 1668*vs*, 1640*m* cm¹.

### Example 14

### 1-(4-Methyl-1,4,4a,5,6,7,8,8a-octahydronaphth-2-yl)pent-4-ene-1-one

### 1-(4-Methyl-3,4,4a,5,6,7,8,8a-octahydronaphth-2-yl)pent-4-ene-1-one

### 1-(4-Methyl-1,2,3,4,5,6,7,8-octahydronaphth-2-yl)pent-4-ene-1-one

Odor (mixture of 3 isomers): woody, green-galbanum, fruity, fatty
¹H-NMR (200MHz, CDCl₃): 6.62 + 6.59 (2*m*, ∼0.8H, C*H*=C), 5.82 (*m*, 1H, C*H*=CH₂), 5.00 (*m*, 2H, CH=C*H*₂), 2.75 (*m*, 2H), 2.68-0.70 (*m*, 18H). MS (EI): Peak 1 (11%): 232(M⁺,4), 217(1), 203(3), 190(8), 177(22), 159(3), 149(100), 133(14), 121(6), 119(8),105(37), 91(39), 81(24), 79(22), 67(15), 55(53), 41(20); Peak 2 (56%): 232(M⁺,42), 217(5), 203(3), 191(33), 177(100), 159(11), 149(75), 135(9), 133(14), 121(16), 109(31), 107(26), 93(40), 91(44), 81(48), 67(57), 55(74), 41(44); Peak 3 (33%): 232(M⁺,78), 217(7), 203(3), 191(73), 177(100), 159(15), 149(32), 135(13), 121(21), 109(57), 107(35), 93(47), 91(43), 81(56), 67(68), 55 (80) , 41(50). IR (mixture of 3 isomers, neat) : 2922*vs*, 2852*s*, 1711*m*, 169*vs*, 1640*m* cm¹.

### Example 15

### 1-(3a,4,5,6,7,7a-hexahydro-1H-indene-2-yl)pent-4-ene-1-one 1-(2,3,4,5,6,7-hexahydro-1H-indene-2-yl)pent-4-ene-1-one

Odor (mixture of 3 isomers): fruity, pineapple, green-galbanum
¹H-NMR (400MHz, CDCl₃): 6.69 (*m*, 0.8H, C*H*=C), 5.83 (*m*, 1H, C*H*=CH₂), 5.05 (*m*, 1H, CH=C*H*₂), 4.98 (*m*, 1H, CH=C*H*₂), 2.75 (*m*, 2H), 2.60-2.20 (*m*, 5H), 1.90 (*m*, 1H), 1.78-0.25 (*m*, 8H). MS (EI): Peak 1 (3%): 204(M⁺,5), 149(3), 121(100), 105(3), 93(20), 91(13), 79(30), 77(12), 67(12), 55(13), 41(6); Peak 2 (16%): 204(M⁺,16), 189(9), 162(4), 149(34), 131(2), 121(100), 107(9), 105(5), 93(18), 91(28), 79(27), 77(14), 67(11), 55(20), 41(9); Peak 3 (79%): 204(M⁺,12), 189(1), 163(14), 149(100), 131(11), 121(47), 107(7), 105(8), 93(29), 91(21), 79 (37) , 77(17), 67(14), 55 (18) , 41(9); IR (mixture of 3 isomers, neat) : 2925*vs*, 2851*s*, 1667*vs*, 1641*m* cm⁻¹.

### Example 16

| Feminine composition for toiletries | |
|---|---|
| | parts per weight |
| Adoxal (10% DPG) | 10 |
| Ambrofix | 3 |
| Beta-ionone | 10 |
| Bergamote oil abergapt | 50 |
| Calone 1951 (10% DPG) | 10 |
| Cepionate | 200 |
| Citronellol | 30 |
| Dasmascenone (10% DPG) | 10 |
| Dihydromyrcenol | 30 |
| Ethyllinalool | 40 |
| Florhydral | 5 |
| Galaxolide 50% PHT | 200 |
| α-Hexyl cinnamic aldehyde | 80 |
| Phenylethyl alcohol | 50 |
| Indol (10% DPG) | 5 |
| Iso E super | 40 |
| Isoraldeine 95 | 50 |
| Jasmone cis (10% DPG) | 10 |
| Lilial | 50 |
| Melonal (10% DPG) | 5 |
| Methyl anthranilate (10% DPG) | 10 |
| Methyl pamplemousse (1,1-dimethoxy-2,2,5-trimethyl-4-hexene) | 20 |
| Nectaryl | 2 |
| Radjanol | 20 |
| Tropional | 15 |
| Tricyclal (10% DPG) | 10 |
| Vanilline (10% DPG) | 5 |
| Viridine (10% DPG) | 10 |
| Ylang Ylang oil. | 10 |
| 2-Cyclohexyl-hepta-1,6-dien-3-one (10% DPG) | 10 |
| | 1000 |

In this feminine accord, 2-cyclohexyl-hepta-1,6-dien-3-one enhances the fruity hesperidic part giving a vibrant character to the fragrance. Its long-lasting effects is very useful to keep the fragrance fresh over time.

### Example 17

| Green-marine composition for toiletries | |
|---|---|
| | parts per weight |
| Acetal CD (phenylacetaldehyde glycerylacetal) | 40 |
| Acetal R (acetaldehyde phenylethyl propyl | 2 |
| acetal) | |
| Adoxal 10% DPG (2,6,10-trimethyl-9-undecenal) | 1.5 |
| Phenoxyethyl alcohol | 70 |
| Linalyl benzoate | 100 |
| Bigarade oil | 10 |
| Clonal (10% DPG) | 5 |
| Beta-damascone | 5 |
| Dimetol (2,6-dimethyl-2-heptanol) | 25 |
| Disopropylene glycol | 375 |
| Florhydral | 20 |
| Glycolierral | 60 |
| (*Z*)-Hex-3-en-1-ol | 5 |
| (Z)-Hex-3-en-1-yl acetate | 2.5 |
| (*Z*)-Hex-3-en-1-yl formiate | 5 |
| (*Z*)-Hex-3-en-1-yl salycilate | 5 |
| Hexyl propionate | 10 |
| Beta-ionone | 10 |
| Linalool | 100 |
| Linalool oxyde (1% DPG) | 2.5 |
| Melonal (10% DPG) | 2.5 |
| Menthe Crepue Ess USA (10% DPG) | 10 |
| Nerol | 80 |
| Nerolidol | 40 |
| Radjanol | 5 |
| Viridine (10% PE) | 5 |
| 2-Cyclohexylhepta-1,6-dien-3-one | 4 |
| | 1000 |

The new compound, namely 2-cyclohexylhepta-1,6-dien-3-one, brings a fresh, natural galbanum note to the perfumed composition, enhancing its diffusion and adding a fresh pineapple top note.

### Example 18

| Floral composition for fabric softener | |
|---|---|
| | parts per weight |
| Acetate PA (2-propenyl phenoxyacetate) | 15 |
| Allyl amyl glycolate | 15 |
| Ambrofix | 1 |
| Benzyl acetate | 60 |
| Citronellol extra | 50 |
| Citronellyl acetate | 30 |
| Cyclal C | 10 |
| Dihydromyrcenol | 50 |
| Ebanol | 10 |
| Freskomenthe | 5 |
| Galaxolide 50% PHT | 60 |
| Geranitrile | 12 |
| Givescone | 7 |
| α-Hexylcinnamic aldehyde | 200 |
| Iso E Super | 30 |
| Linalool | 60 |
| Lilial | 60 |
| Nectaryl | 5 |
| Neroline crist. | 5 |
| Rosacetol | 20 |
| Roseoxyde | 8 |
| Terpineol | 60 |
| 10-Undecenal | 2 |
| Verdyl acetate | 200 |
| Ylang Oil | 15 |
| 2-Cyclohexylhepa-1,6-dien-3-one (10% DPG) | 10 |
| | 1000 |

In this floral accord 2-cyclohexylhepta-1,6-dien-3-one with its green-galbanum note gives a fresh and clean effect to the fragrance, both on wet and on dry laundry.

### Example 19

| Fresh floral composition for softeners | |
|---|---|
| | parts per weight |
| Adoxal (10% DPG) | 1 |
| Agrumex | 40 |
| Amberketal (10% IPM) | 15 |
| Ambretone | 10 |
| Bigarade oil | 20 |
| Citronellol | 35 |
| Beta-Damascene | 2.5 |
| Dimethylbenzylcarbinyl acetate | 75 |
| Ebanol | 20 |
| Fixambrene | 10 |
| Florhydral | 10 |
| Gardenol | 7.5 |
| α-Hexylcinnamic aldehyde | 70 |
| Hexyl salicylate | 100 |
| Beta-ionone | 40 |
| Isoraldeine | 30 |
| Jasmin reconst. | 15 |
| Jasmonyl | 25 |
| Lilial | 70 |
| 2-Methylundecanal | 5 |
| Oranger crystals | 4 |
| Phenylethylalcohol | 85 |
| Rosacetol | 30 |
| Tetrahydrolinalool | 100 |
| Thibetolide | 20 |
| Undecavertol | 25 |
| 9-Undecenal | 5 |
| Verdylpropionate | 55 |
| Vertofix coeur | 55 |
| 1-(1,4,4a,5,6,7,8,8a-Octahydronaphth-2-yl)pent-4-ene-1-one | 20 |
| 1-(3,4,4a,5,6,7,8,8a-Octahydronaphth-2-yl)pent-4-ene-1-one | |
| | 1000 |

The novel compound adds a fresh, fruity galbanum note to the perfumed composition, enhancing its volume, diffusion and long-lastingness.

For the exact definition of the trivial names mentioned above, see Flavor and Fragrance materials 1998, Allured publishing Corporation, Carol Stream, Illinois, U.S.A. or Arctander, Perfume and Flavor Chemicals - 1969, published by the author, Montclair, New Jersey, U.S.A.

## Claims

1. Compounds of the general formula I 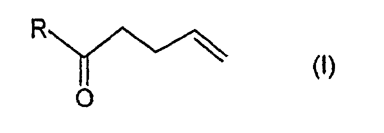 wherein R is a residue A 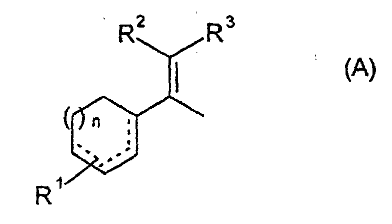 or a residue B 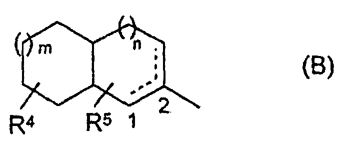 and R¹, R², R³, R⁴ and R⁵ are independently hydrogen, methyl or ethyl, R¹, R⁴ and R⁵ being at any position of the ring structure, n and m are independently 0,1,2 or 3 and the dotted lines stand in formula A, if desired, for a double bond and the dotted lines in formula B stand for a double bond either in position 1 or 2.

2. Compound according to claim 1 of the general formula Ia 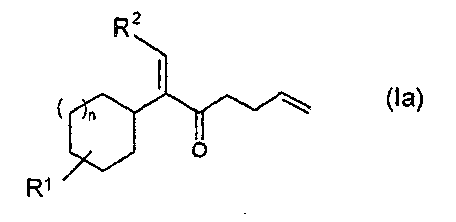 wherein R¹ and R² are independently hydrogen or methyl and n stands for 0 or 1.

3. 2-Cyclohexylhepta-1,6-dien-3-one according to claim 1.

4. (E)- and (Z)-3-Cyclohexylocta-2,7-dien-4-one according to claim 1.

5. 2-Cyclopentylhepta-1,6-dien-3-one according to claim 1.

6. 1-(3,4,4a,5,6,7,8,8a-Octahydronaphth-2-yl)pent-4-ene-1-one according to claim 1.

7. 1-(1,4,4a,5,6,7,8,8a-Octahydronaphth-2-yl)pent-4-ene-1-one according to claim 1.

8. Mixture of 1-(3,4,4a,5,6,7,8,8a-Octahydronaphth-2-yl)pent-4-ene-1-one and 1-(1,4,4a,5,6,7,8,8a-Octahydronaphth-2-yl)pent-4-ene-1-one according to claim 1.

9. Odoriferous composition comprising at least one compound of formula I according to one of the preceding claims.

10. Composition according to claim 9 exhibiting a green-galbanum fresh (metallic type) and fruity-pineapple and/or fruity-cassis odor.

## Patentansprüche

1. Verbindungen der allgemeinen Formel I, 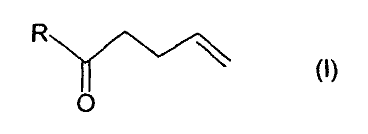 in der R ein Rest A 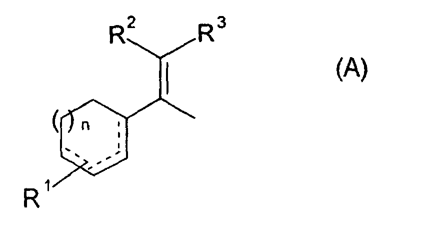 oder ein Rest B ist, 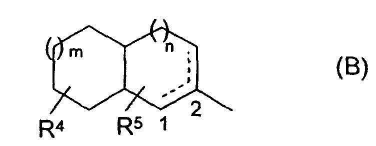 und R¹, R², R³, R⁴ und R⁵ unabhängig voneinander Wasserstoff, Methyl oder Ethyl sind, wobei R¹, R⁴ und R⁵ an irgendeiner Position des Rings sind, n und m unabhängig voneinander 0, 1, 2 oder 3 sind, und die gestrichelten Linien in Formel A, wenn erwünscht, für eine Doppelbindung stehen, und die gestrichelten Linien in Formel B für eine Doppelbindung entweder in Position 1 oder 2 stehen.

2. Verbindung nach Anspruch 1 der allgemeinen Formel Ia, 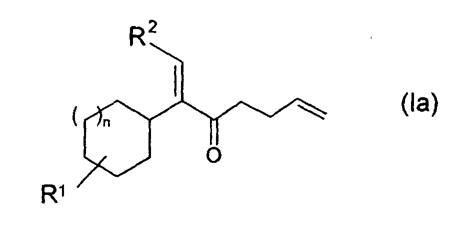 in der R¹ und R² unabhängig voneinander Wasserstoff oder Methyl sind, und n für 0 oder 1 steht.

3. 2-Cyclohexylhepta-1,6-dien-3-on nach Anspruch 1.

4. (E)- und (Z)-3-Cyclohexylocta-2,7-dien-4-on nach Anspruch 1.

5. 2-Cyclopentylhepta-1,6-dien-3-on nach Anspruch 1.

6. 1-(3,4,4a,5,6,7,8,8a-Octahydronaphth-2-yl)pent-4-en-1-on nach Anspruch 1.

7. 1-(1,4,4a,5,6,7,8,8a-Octahydronaphth-2-yl)pent-4-en-1-on nach Anspruch 1.

8. Mischung von 1-(3,4,4a,5,6,7,8,8a-Octahydronaphth-2-yl)pent-4-en-1-on und 1-(1,4,4a,5,6,7,8,8a-Octahydronaphth-2-yl)pent-4-en-1-on nach Anspruch 1.

9. Duftende Zusammensetzung enthaltend mindestens eine Verbindung der Formel I nach einem der vorangehenden Ansprüche.

10. Zusammensetzung nach Anspruch 9, die einen grün-Galbanum-frischen (metallartigen) und fruchtig-Ananas- und/oder fruchtig-Cassis-Duft verbreitet.

## Revendications

1. Composé de formule générale I 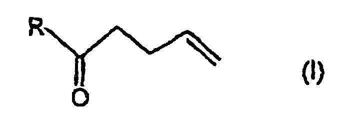 dans laquelle R représente un résidu A 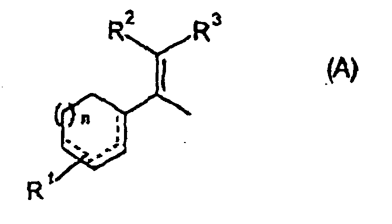 ou un résidu B 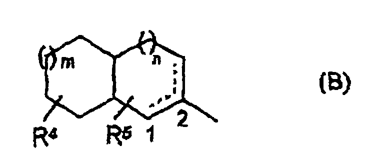 et R¹, R², R³, R⁴ et R⁵ représentent indépendamment un atome d'hydrogène, un groupe méthyle ou éthyle, R¹, R⁴ et R⁵ se trouvant en une position quelconque de la structure du cycle, n et m valant indépendamment 0, 1, 2 ou 3 et les lignes pointillées représentent dans la formule A, si souhaité, une double liaison, et les lignes pointillées dans la formule B représentent une double liaison en position 1 ou 2.

2. Composé selon la revendication 1 de formule générale Ia 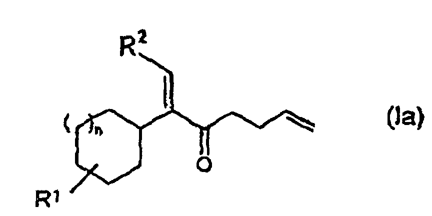 dans laquelle R¹ et R² représentent indépendamment un atome d'hydrogène ou un groupe méthyle et n vaut 0 ou 1.

3. 2-Cyclohexylhepta-1,6-dièn-3-one selon la revendication 1.

4. (E)- et (Z)-3-Cyclohexylocta-2,7-dièn-4-one selon la revendication 1.

5. 2-Cyclopentylhepta-1,6-dièn-3-one selon la revendication 1.

6. 1-(3,4,4a,5,6,7,8,8a-Octahydronapht-2-yl)pent-4-ène-1-one selon la revendication 1.

7. 1-(1,4,4a,5,6,7,8,8a-Octahydronaphth-2-yl)pent-4-ène-1-one selon la revendication 1.

8. Mélange de 1-(3,4,4a,5,6,7,8,8a-Octahydronapht-2-yl)pent-4-ène-1-one et de1-(1,4,4a,5,6,7,8,8a-Octahydronapht-2-yl)pent-4-ène-1-one selon la revendication 1.

9. Composition odoriférante comprenant au moins un composé de formule I selon l'une quelconque des revendications précédentes.

10. Composition selon la revendication 9, présentant une odeur fraîche verte de galbanum (de type métallique) et de fruitée d'ananas et/ou fruitée de cassis.
